# EUROPEAN PATENT APPLICATION

(11) **EP 3 219 785 A1**
(43) Date of publication of application: **20.09.2017**
(21) Application number: 15859223.8
(22) Date of filing: 09.11.2015
(51) Int. Cl.: C12M 1/42, C12M 3/00, C01B 32/182, C23C 14/06

(54) **CELL SHEET MANUFACTURING DEVICE AND MANUFACTURING METHOD THEREFOR**

(30) Priority: 11.11.2014 KR 20140155910
(71) Applicant: Seoul National University R&DB Foundation, Gwanak-gu Seoul 08826 (KR); Institute for Basic Science, Daejeon 34047 (KR)
(72) Inventor: KIM, Daehyeong, Incheon 21986 (KR); CHOI, Seunghong, Seoul 06281 (KR); HYEON, Taeghwan, Seoul 06277 (KR); KIM, Seokjoo, Seoul 05275 (KR); CHO, Hyerim, Seoul 04736 (KR); CHO, Kyoungwon, Goyang-si Gyeonggi-do 10322 (KR)
(74) Representative: Ter Meer Steinmeister & Partner
(86) International application number: PCT/KR2015/012020
(87) International publication number: WO 2016/076590

(57) **Abstract**

The present invention relates to a cell sheet manufacturing device and a manufacturing method therefor. More specifically, the present invention relates to a cell sheet manufacturing device comprising a support layer made of silicon rubber, a patterned electrode formed adjacent to the support layer and a graphene layer formed adjacent to the electrode, and a manufacturing method therefor.

## Description

### Technical Field

The present invention relates to a cell sheet manufacturing device and a manufacturing method therefor. More specifically, the present invention relates to a cell sheet manufacturing device comprising a support layer made of silicon rubber, a patterned electrode formed adjacent to the support layer and a graphene layer formed adjacent to the electrode, and a manufacturing method therefor.

### Background Art

Tissue engineering scaffolds serve as synthetic extracellular matrices (ECMs) to support cells in culture systems and to assist the regeneration of damaged tissues (Levenberg, S. et al. Differentiation of human embryonic stem cells on three-dimensional polymer scaffolds. Proc. Natl. Acad. Sci. U. S. A. 100, 12741-12746 (2003)). Although significant progress in scaffold technology has been made (Dvir, T., Timko, B.P., Kohane, D.S. & Langer, R. Nanotechnological strategies for engineering complex tissues. Nat. Nanotechnol. 6, 13-22 (2011), Sun, J.Y. et al. Highly stretchable and tough hydrogels. Nature 489, 133-136 (2012)), including improvements in materials and structural designs, most scaffolds remain passive; that is, they provide only structural support without any in situ monitoring capability. Passive scaffolds thus need ex situ biological assays which demands death or sacrifice of cells.

Therefore, active scaffolds embedded with sensors that continuously monitor cellular physiology and environment correspond to a new frontier of tissue engineering (Tian, B. et al. Macroporous nanowire nanoelectronic scaffolds for synthetic tissues. Nat. Mater. 11, 986-994 (2012)).

Wafer-based electronics have demonstrated such capabilities (Straub, B., Meyer, E. & Fromherz, P. Recombinant maxi-K channels on transistor, a prototype of iono-electronic interfacing. Nat. Biotechnol. 19, 121-124 (2001)); however, these rigid, planar wafers are mechanically incompatible with flexible tissues. Flexible (Sekitani, T., Zschieschang, U., Klauk, H. & Someya, T. Flexible organic transistors and circuits with extreme bending stability. Nat. Mater. 9, 1015-1022 (2010), Viventi, J. et al. A conformal, bio-interfaced class of silicon electronics for mapping cardiac electrophysiology. Sci. Transl. Med. 2, 24ra22 (2010), Viventi, J. et al. Flexible, foldable, actively multiplexed, high-density electrode array for mapping brain activity in vivo. Nat. Neurosci. 14, 1599-1605 (2011), Kaltenbrunner, M. et al. An ultra-lightweight design for imperceptible plastic electronics. Nature 499, 458-463 (2013)) and stretchable (Sekitani, T. et al. A rubberlike stretchable active matrix using elastic conductors. Science 16 321, 1468-1472 (2008), Kim, D.H. et al. Epidermal Electronics. Science 333, 838-843 (2011), Keplinger, C. et al. Stretchable, transparent, ionic conductors. Science 341, 984-987 (2013), Son, D. et al. Multifunctional wearable devices for diagnosis and therapy of movement disorders. Nat. Nanotechnol. 9, 397-404 (2014)) electronics can offer a viable solution toward this purpose, .

Meanwhile, biodegradability and cytotoxicity of onboard electronic devices may hinder the potential in vivo implantation of such electronic scaffolds. Therefore, scaffold-free cell sheet treatment is an attractive approach to overcome these limitations. Cell sheet therapy provides robust benefits in localized treatments by eliciting high paracrine effects and has been successfully used in the cornea (Nishida, K. et al. Corneal reconstruction with tissue-engineered cell sheets composed of autologous oral mucosal epithelium. N. Engl. J. Med. 351, 1187-1196 (2004)), periodontium (Iwata, T. et al. Periodontal regeneration with multi-layered periodontal ligament-derived cell sheets in a canine model. Biomaterials 30, 2716-2723 (2009)) and heart (Miyahara, Y. et al. Monolayered mesenchymal stem cells repair scarred myocardium after myocardial infarction. Nat. Med. 12, 459-465 (2006)).

A temperature-sensitive polymer, poly(N-isopropylacrylamide) (PIPAAm), has been widely used to prepare and transfer cell sheets to in vivo models (Haraguchi, Y. et al. Fabrication of functional three-dimensional tissues by stacking cell sheets in vitro. Nat. Protoc. 7, 850-858 (2012)).

However, the electron irradiation process required to precisely control the ultrathin thickness (<30 nm) of the PIPAAm substrate (Akiyama, Y., Kikuchi, A., Yamato, M. & Okano, T. Ultrathin poly(Nisopropylacrylamide) grafted layer on polystyrene surfaces for cell adhesion/detachment control. Langmuir 20, 5506-5511 (2004)) is highly delicate. Moreover, integrating PIPAAm with inorganic electronics is complicate because of its instability during the photolithography process. Therefore, a simpler and more effective method for scaffold-free cell sheet treatment is needed.

### Disclosure

### Technical Problem

The basic object of the present invention is to provide a cell sheet manufacturing device comprising a support layer, a patterned electrode formed adjacent to the support layer and a graphene layer formed adjacent to the electrode.

Another object of the present invention is to provide a manufacturing method of a cell sheet manufacturing device comprising (i) forming a nickel layer on a silicon wafer; (ii) forming an electrode layer on the nickel layer in order; (iii) patterning the electrode layer; (iv) forming a graphene layer on the patterned electrode layer; (v) forming an adhesion layer on the graphene layer; (vi) separating the silicon wafer by etching and removing the nickel layer; (vii) attaching the separated adhesion layer/graphene layer/electrode layer on silicon rubber layer; and (viii) removing the adhesion layer.

Another object of the present invention is to provide a manufacturing method of a cell sheet comprising culturing cells on a cell sheet manufacturing device which comprises a support layer, a patterned electrode formed adjacent to the support layer and a graphene layer formed adjacent to the electrode.

### Technical Solution

The object of the present invention can be achieved by providing a cell sheet manufacturing device comprising a support layer, a patterned electrode formed adjacent to the support layer and a graphene layer formed adjacent to the electrode.

The support layer may be formed of silicon rubber, glass, PET film or wafer. The silicon rubber may be polydimethylsiloxane. Polydimethylsiloxane is flexible and stretchable, and is effective in the transfer printing of the cell sheet since it has system modulus suitable for the culture of cell layers.

In addition, the electrode may be an electrode selected from a group consisting of Al, Cu, Pt and Cr/Au. Preferably, the electrode may be Cr/Au electrode. For example, metals that can be deposited by thermal evaporator or e-beam evaporator can be used as materials for the electrode. In one embodiment of the present invention, since adhesive strength of Au with the substrate is not strong, Au can be re-deposited on Cr thin film after thinly depositing Cr with a good adhesive strength with Au on the substrate.

In addition, the graphene layer may be a patterned layer. In one embodiment of the present invention, since muscle cells are aligned inside body, if the graphene is patterned, the muscle cells proliferate directionally in one direction and thus it is suitable to culture the muscle cells. For example, if patterning the graphene to be arranged in one direction at an interval of 5 *µ*m or less, when the muscle cells are cultured thereon, the muscle cells proliferate directionally in one direction.

Moreover, a state of cell growth and proliferation can be checked in real time from an electric signal obtained through the electrode. In one embodiment of the present invention, impedance can be obtained by an alternating current that flows through the culture medium between two electrodes spaced apart from each other, and conductance can be obtained by a direct current. If cells are cultured on the electrode, cells change the current flow and thus results of impedance and conductance are changed. The state change of cells can be checked by measuring the change of impedance and conductance depending on proliferation and differentiation of cells in real time based on this principle. Next, the temperature change can be checked as follows. In case of metals, their resistance changed according to the temperature. Therefore, if measuring the resistance, temperature signals around the electrode can be found. There is an advantage that it is possible to measure the change of the temperature environment in real time when culturing cells by using this temperature sensor.

In one embodiment of the present invention, in order to treat and regenerate cells (for example, muscle cells) using the cell sheet of the present invention, first, the transfer printing process for moving the cell sheet is needed. The next two steps are needed to conduct the transfer printing using the substrate: i) (Preparation step) Taking out the device where the cell sheet is attached from cell culture medium, and placing it upside down on the plastic substrate (in order that cell sheet and plastic substrate are in contact). If detaching the device about 30 seconds later, the cell sheet is separated from on the plastic substrate with the cell sheet attached to the device. ii) (Transfer step) Placing the device that passed through the preparation step and the cell sheet was attached to upside down on the desired target tissue (in order that cell sheet and tissue are in contact). In order to be tightly attached, PDMS rubber is pressed gently to remove bubbles. If detaching the device about 90 seconds later, the cell sheet is attached to the target tissue to remain and the remnant device is separated. By performing the two steps repeatedly, the transfer printing of multi-layered cell sheet to in vivo tissue can be performed in 3D structure. The transplanted cell sheet proceeds to proliferation and differentiation in vivo, secretes various substances urging the regeneration of peripheral damaged tissue to directly/indirectly affect the regeneration of damaged tissue.

Another object of the present invention can be achieved by providing a manufacturing method of a cell sheet manufacturing device comprising (i) forming a nickel layer on a silicon wafer; (ii) forming an electrode layer on the nickel layer in order; (iii) patterning the electrode layer; (iv) forming a graphene layer on the patterned electrode layer; (v) forming an adhesion layer on the graphene layer; (vi) separating the silicon wafer by etching and removing the nickel layer; (vii) attaching the separated adhesion layer/graphene layer/electrode layer on silicon rubber layer; and (viii) removing the adhesion layer.

In the manufacturing method of a cell sheet manufacturing device of the present invention, the electrode layer may be an electrode layer selected from a group consisting of Al, Cu, Pt and Cr/Au. Preferably, the electrode layer may be Cr/Au electrode. For example, metals that can be deposited by thermal evaporator or e-beam evaporator can be used as materials for the electrode. In one embodiment of the present invention, since adhesive strength of Au with the substrate is not strong, Au can be re-deposited on Cr thin film after thinly depositing Cr with a good adhesive strength with Au on the substrate.

In addition, in the manufacturing method of a cell sheet manufacturing device of the present invention, the step of patterning the graphene layer may be added before the step (v). In one embodiment of the present invention, since muscle cells are aligned inside the body, if the graphene is patterned, the muscle cells proliferate directionally in one direction and thus it is suitable to culture the muscle cells. For example, if patterning the graphene to be arranged in one direction at an interval of 5 *µ*m or less, when the muscle cells are cultured thereon, the muscle cells proliferate directionally in one direction.

Moreover, the adhesion layer may be PMMA. More specifically, the PMMA may be PMMA A2 or PMMA A4.

Another object of the present invention can be achieved by providing a manufacturing method of a cell sheet comprising culturing cells on a cell sheet manufacturing device which comprises a support layer, a patterned electrode formed adjacent to the support layer and a graphene layer formed adjacent to the electrode.

In the manufacturing method of a cell sheet of the present invention, the support layer may be formed of silicon rubber, glass, PET film or wafer. The silicon rubber may be polydimethylsiloxane.

In addition, the electrode may be an electrode selected from a group consisting of Al, Cu, Pt and Cr/Au. Preferably, the electrode layer may be Cr/Au electrode. For example, metals that can be deposited by thermal evaporator or e-beam evaporator can be used as materials for the electrode. In one embodiment of the present invention, since adhesive strength of Au with the substrate is not strong, Au can be re-deposited on Cr thin film after thinly depositing Cr with a good adhesive strength with Au on the substrate.

In addition, the graphene layer may be a patterned layer. In one embodiment of the present invention, since muscle cells are aligned inside the body, if the graphene is patterned, the muscle cells proliferate directionally in one direction and thus it is suitable to culture the muscle cells. For example, if patterning the graphene to be arranged in one direction at an interval of 5 *µ*m or less, when the muscle cells are cultured thereon, the muscle cells proliferate directionally in one direction.

Moreover, a state of cell growth and proliferation can be checked in real time from an electric signal obtained through the electrode. In one embodiment of the present invention, impedance can be obtained by an alternating current that flows through the culture medium between two electrodes spaced apart from each other, and conductance can be obtained by a direct current. If cells are cultured on the electrode, cells change the current flow and thus results of impedance and conductance are changed. The state change of cells can be checked by measuring the change of impedance and conductance depending on proliferation and differentiation of cells in real time based on this principle. Next, the temperature change can be checked as follows. In case of metals, their resistance changed according to the temperature. Therefore, if measuring the resistance, temperature signals around the electrode can be found. There is an advantageous that it is possible to measure the change of the temperature environment in real time when culturing cells by using this temperature sensor.

### Advantageous Effects

By fabricating the cell sheet using the device of the present invention, the physiological change of cells which could be detected through various complicate methods can be detected easily and in real time by electrical signals, and this real time measuring method guarantees the ease and accuracy of the experiment. Moreover, by measuring electrical signals, there is an advantage to block cellular loss resulting from prior experiment methods.

In addition, since it is possible to perform the transfer printing of the cell sheet under at optimal state by observing cells state in real time, the treatment effect can be maximized. The regeneration of damaged tissues can be maximized by performing the transfer printing of the activated cell sheet fabricated in this way to the desired body tissue through newly developed transfer printing method with high yield near 100 %.

### Description of Drawings

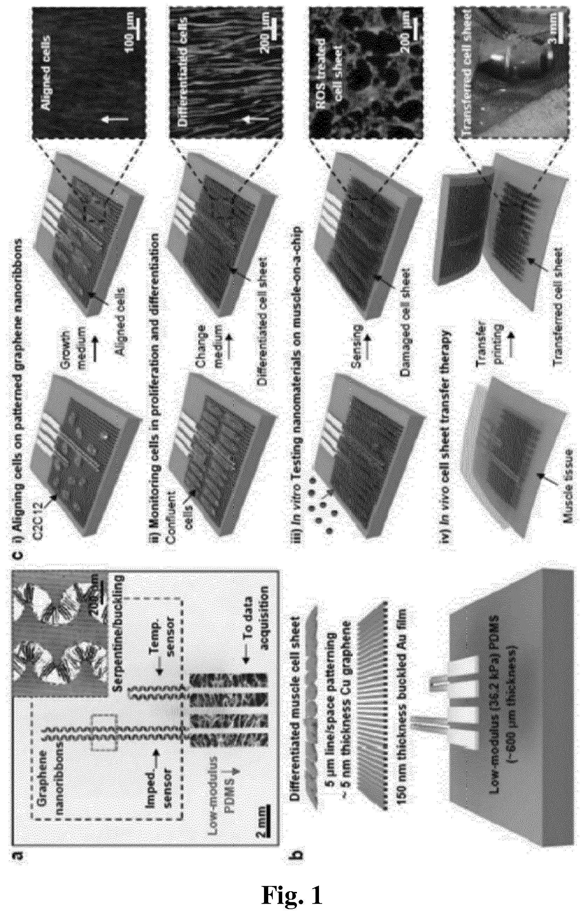

Fig. 1a is a cell-culture platform according to a present invention (the inset at the top right position shows the magnified view of serpentine-shaped buckled Au electrodes). The cell-culture platform consists of patterned graphene nanoribbons (top layer) and Au nanomembrane impedance/temperature sensors (bottom layer) on a low-modulus PDMS sheet. Fig. 1b is an expanded view of each element of the cell-culture platform. C2C12 cells are cultured on the patterned graphene nanoribbons. Fig. 1c shows the four key applications of the soft instrumented cell-culture platform according to a present invention, that is, (i) alignment of C2C12 myoblasts, (ii) in situ monitoring of proliferation and differentiation, (iii) evaluating the effects of novel nanomaterials and drugs in vitro, and (iv) transfer printing of cultured cell sheets onto target sites of animal models in vivo.

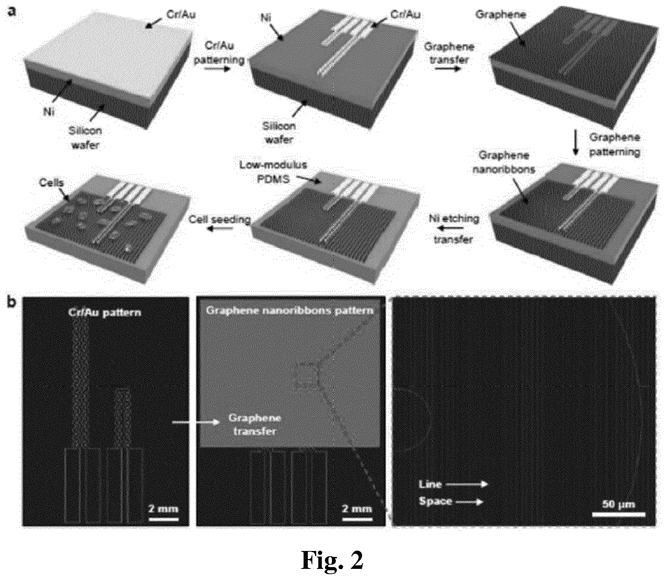

Fig. 2a is a schematic view of the fabrication process of a soft cell-culture platform according to a present invention. Fig. 2b shows AutoCAD designs of impedance and temperature sensors along with the patterned graphene nanoribbons.

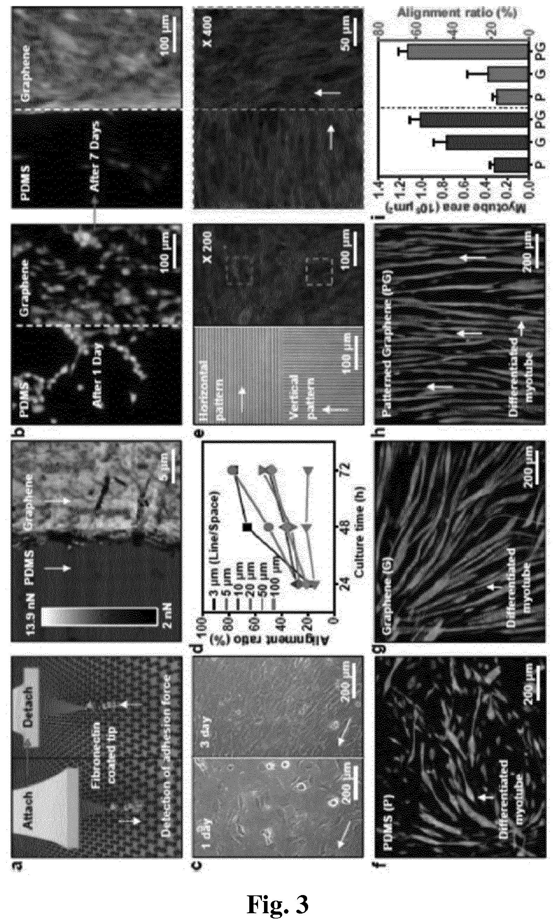

Fig. 3a shows the measurement of the adhesion force between fibronectin molecules and graphene (The left frame shows a schematic illustration of AFM adhesion force measurements using the fibronectin-coated AFM tip, and the right frame represents the force map). Fig. 3b is images showing the biocompatible, cell-friendly surface of patterned graphene nanoribbons. Fig. 3c is images of aligned C2C12 myoblasts on the patterned graphene nanoribbons (about 5 *µ*m widths of both lines and spaces). Fig. 3d shows cell alignment ratio as a function of culture time for different graphene feature sizes (The smaller feature size (about 5 *µ*m widths of both lines and spaces) yields a better alignment than the larger feature size). Fig. 3e shows cell alignment on patterned graphene where horizontal (top) and vertical (bottom) patterns coexist (left frame), and magnified images of red- and blue-dotted boxed regions (right frame). Figs. 3f to 3h show myotube formation through myogenic differentiation after 7 days of culture on different substrates, including PDMS(P), graphene (G) and patterned graphene (PG) (The white arrows indicate the direction of myotube alignment). Fig. 3i shows the plot of the myotube areas and alignment ratios on different substrates.

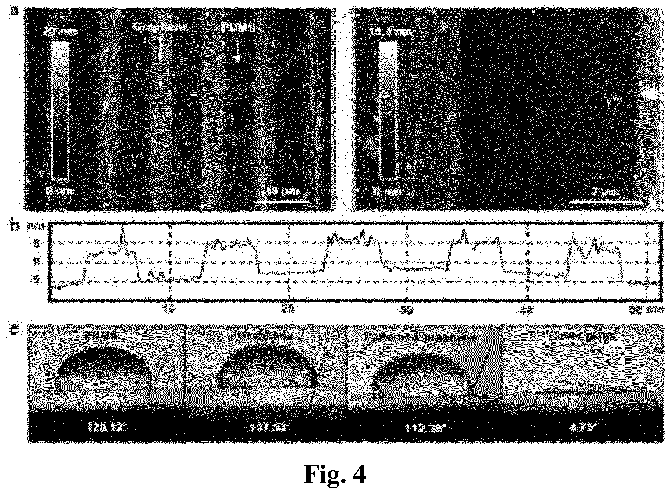

Fig. 4a is AFM topological image of the patterned graphene on PDMS (Spring coefficient of cantilevers (TESP, Bruker probes) is 42 N/m and all AFM data are measured with a tapping mode, and data are visualized by the NanoScope Analysis software (Bruker)). Fig. 4b is AFM topological graph of patterned graphene on PDMS. Fig. 4c shows water contact angles of various substrates: PDMS, graphene, patterned graphene (widths of line and spacing; 5 *µ*m), and cover glass.

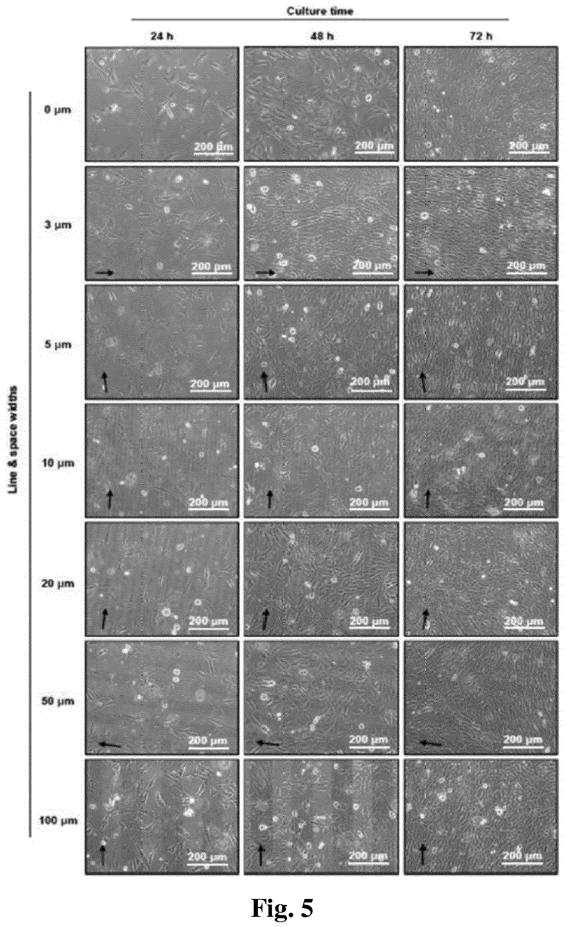

Fig. 5 is phase-contrast microscope images of cell alignments in respect to pattern sizes and culture time. In Fig. 5, C2C12 myoblasts are cultured on 7 different graphene patterns with different line and space widths (0, 3, 5, 10, 20, 50, and 100 *µ*m) to find the best pattern width for cell alignments, and each sample is observed at different time (24, 48, and 72 hours after the culture) by the phase-contrast microscopy.

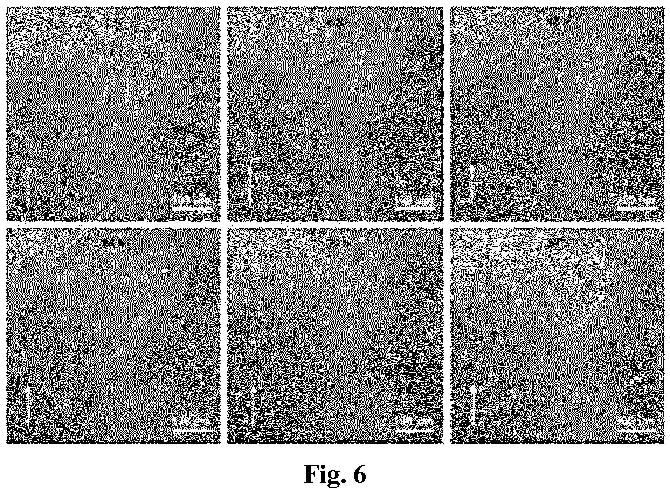

Fig. 6 shows results of real-time monitoring of C2C12 myoblasts on graphene nanoribbons with 5 *µ*m lines and spaces.

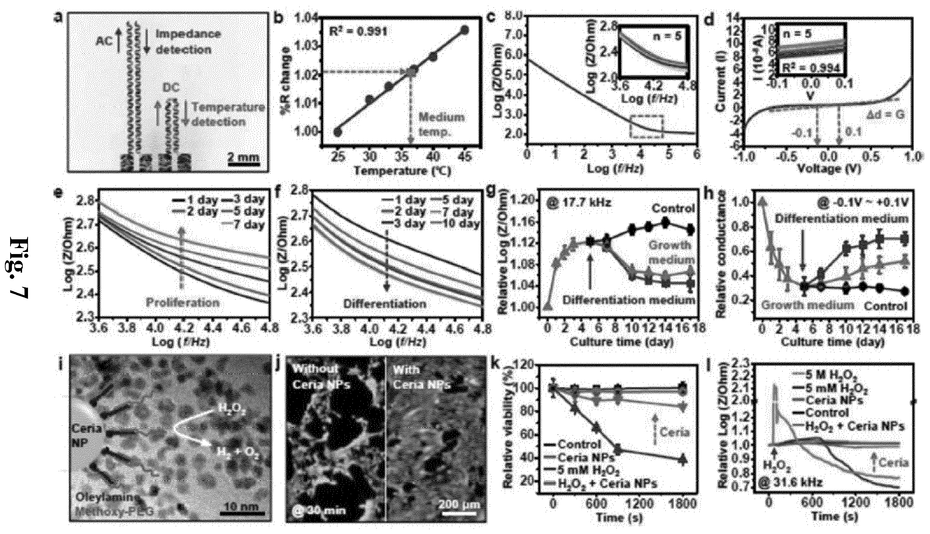

Fig. 7a shows impedance and temperature sensors integrated on a PDMS substrate. Fig. 7b shows calibration curve of temperature sensor: normalized resistance (% R change) as a function of temperature. The red arrow indicates the temperature of the growth medium during the culture. Fig. 7c shows the electrical characteristic of the impedance sensor in the growth medium at 37°C (Impedance curve was measured from 1 Hz to 1 MHz with a bias voltage of 0.01 V. The inset shows the magnified view of the red-dotted region.). Fig. 7d is current-voltage (I-V) curve whose slope indicates the conductance (The inset shows the magnified view. Repeated measurements confirmed the stability of the sensor.). The impedance curve is changed as the proliferation (Fig. 7e) and the differentiation (Fig. 7f) proceeded. Fig. 7g shows impedance value measured at 17.7 kHz as the culture proceeded (Red and blue curves show the cells in growth and differentiation media, respectively. Human dermal fibroblasts are used as the control (black).). Fig. 7h is conductance values calculated from I-V curves with a range from -0.1 to +0.1 V. Fig. 7i is schematic illustration and TEM image (background image) of ROS-scavenging ceria nanoparticles (The ceria nanoparticles (Ceria NPs) were functionalized by oleylamine and methoxy-PEG.). Fig. 7j is fluorescence image of C2C12 myoblasts (stained with calcein AM) after the H₂O₂/Ceria NP treatment for 30 minutes. Fig. 7k is the relative viability plot from fluorescence images. Fig. 7l is the plots of impedance as a function of time in different treatment groups.

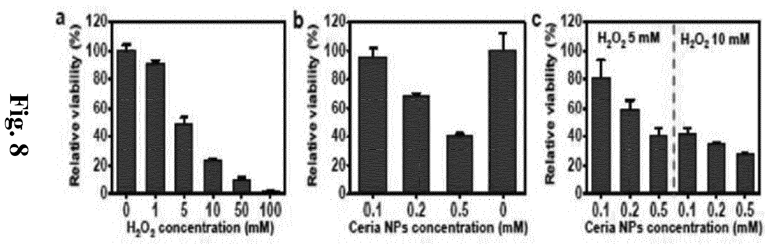

Figs. 8a and 8b are a plot of relative viability of cells depending on the concentration of hydrogen peroxide (H₂O₂) and ceria nanoparticles (Ceria NPs). Fig. 8c is a graph of relative viability of cells versus concentrations of Ceria NPs with 5 mM and 10 mM of H₂O₂ treatment. Optimized ROS concentration is decided when the number of the H₂O₂ treated C2C12 myoblasts decreases by 50% within 30 minutes. After H₂O₂ and Ceria NPs treatment, the samples were incubated in the MTT solution (Amresco) for 3 hours. Data were quantified by using a microplate reader (SpectraMax M3, Moelcular Devices).

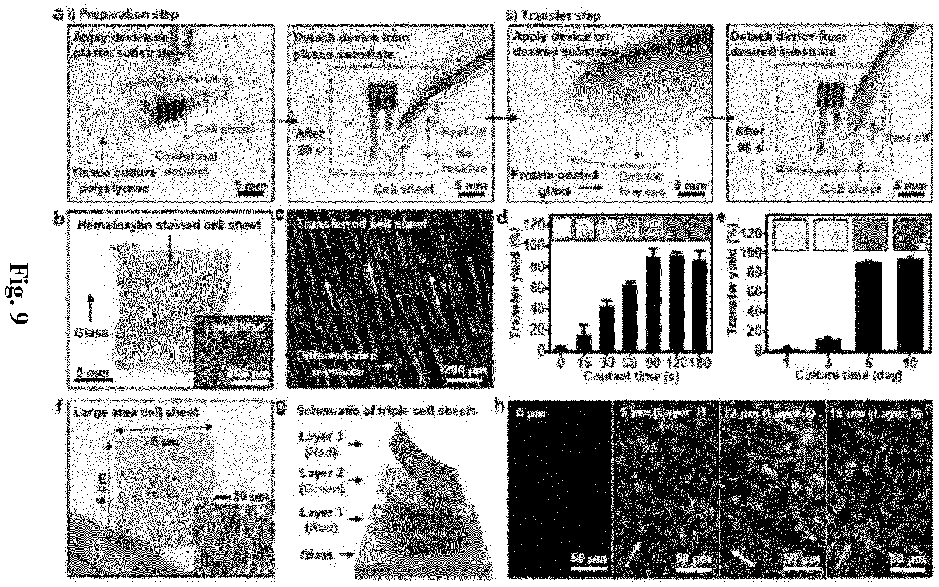

Fig. 9a is images of the cell sheet transfer printing process. Fig. 9b is image of the transferred cell sheet stained with hematoxylin on the protein-coated glass substrate (The inset shows its live/dead viability assay image, stained with calcein AM (green) and ethidium homodimer-1 (red).). Fig. 9c shows that the myosin heavy chains were maintained even after the cell sheet transfer (The white arrows indicate the aligned orientation.). Figs. 9d and 9e are plots of the transfer yields of cell sheets as a function of contact time in the transfer step or as a function of culture time, respectively (The insets show images of transferred cell sheets.). Fig. 9f is image of a 5 × 5 cm region of a hematoxylin-stained cell sheet and its magnified view (inset). Fig. 9g is schematic diagram of three cell sheets transferred layer by layer with orthogonal orientations. Fig. 9h is confocal images of the three-layered cell sheets (Images were captured at a depth interval of 6 *µ*m. The white arrows indicate the direction of cell alignments.).

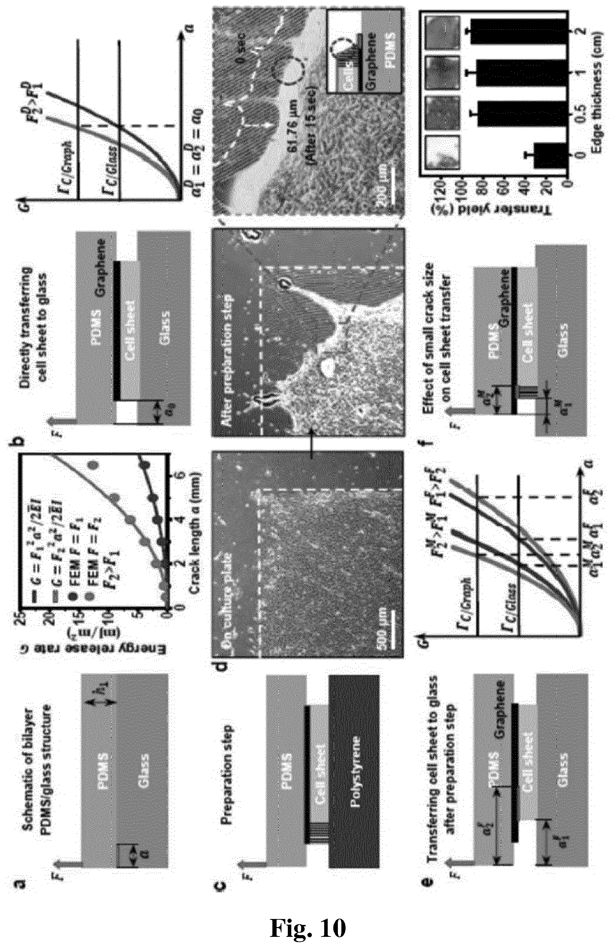

Fig. 10a is analytical (curve) and FEM (dot) results of the energy release rate G versus crack length a under two different peeling forces. Fig. 10b shows that, when directly transferring a cell sheet onto protein-coated glass without the preparation step, the critical force to propagate a crack along the cell-glass interface is lower than that along the cell-graphene interface and thus the adhesion of the cell graphene is stronger. Fig. 10c shows that, during the preparation step, the edge of the cell sheet was damaged due to stress concentration at the crack tip while peeling the PDMS layer. Fig. 10d shows that the damage in the cell sheet is visible under phase-contrast microscopy after the preparation step. Fig. 10e shows that, during the final transfer step, a relatively long cell-graphene interface defect introduced during the preparation step drops the critical force to propagate a crack along this interface when comparing with the cell-glass interface. Fig. 10f shows that, if there is no PDMS margin, i.e., the whole PDMS surface is covered by graphene, the cell-graphene interface defect zone is much smaller, and hence the force to propagate a crack along the cell-graphene interface is not lowered enough to facilitate successful transfer.

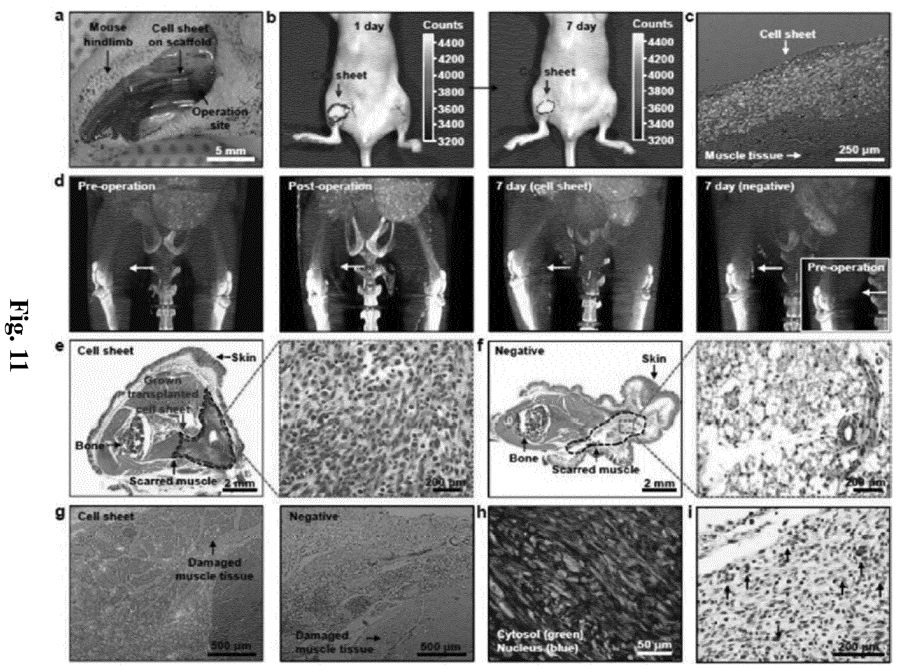

Fig. 11a is image of cell sheet transfer printing applied to scarred hindlimb muscle in a mouse. Fig. 11b is in vivo fluorescence imaging at 1 day (left) and 7 days (right) after transfer printing of the five-layered C2C12 cell sheets labeled with cell tracers (DiD). Fig. 11c show the proliferation of transfer-printed cells (GFP-expressing C2C12 myoblasts) at the operation site 7 days after transplantation. Fig. 11d is CT images of the operated mice: (from left to right) preoperation, postoperation, 7 days after cell sheet transplantation (five-layered), and negative control (no cell sheet transplantation). The white arrows indicate the operation site. The inset shows the preoperation image of the negative experiment group. Figs. 11e and 11f are Hematoxylin and eosin staining results of the operated hindlimb 7 days after transplantation of the cell sheet (five-layered) and negative control without the cell sheet transplantation, respectively, for morphological observations (The red-dotted boxes show magnified views.). Fig. 11g shows the proliferation of the cell sheet around the damaged muscles, as shown by expression of GFP. Fig. 11h shows the differentiation of the cell sheet around the damaged muscles. Fig. 11i shows the vascularization (black arrows) in the region of transferred cell sheets 7 days after transplantation.

### Best Mode

Hereinafter, a detailed description will be given of the present invention with reference to the following embodiments. The purposes, features, and advantages of the present invention will be easily understood through the following embodiments. The present invention is not limited to such embodiments, but may be modified in other forms. The embodiments to be described below are nothing but the ones provided to bring the disclosure of the present invention to perfection and assist those skilled in the art to completely understand the present invention. Therefore, the following embodiments are not to be construed as limiting the present invention.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present invention.

It will be understood that when an element is referred to as being "on" another element, it can be directly on the other element or intervening elements may be present therebetween. In contrast, when an element is referred to as being "directly on" another element, there are no intervening elements present.

### Example1. Fabrication of the soft instrumented cell-culture platform

Graphene was synthesized using the chemical vapor deposition growth process (Li, X. et al. Large-area synthesis of high-quality and uniform graphene films on copper foils. Science 324, 1312-1314 (2009)), and was grown on copper foil (Alfa Aesar, cut in 6cm x 6cm section) at 1000°C with a mixture of methane (20 sccm, 1.6 Torr) and hydrogen (8.4 sccm, 0.8 Torr). The fabrication process of the electronic support body (current cell-culture platform) and the AutoCAD design of the sensors are provided in Figs. 2a and 2b. Ni (30 nm) as a sacrificial layer was deposited onto a Si wafer (test grade, 4Scinece) by thermal evaporation. Next, 7 nm of Cr (adhesion layer) and 150 nm of Au were deposited onto the Ni-deposited Si wafer by thermal evaporation and patterned by photolithography and wet etching. Then, the graphene was transferred onto the sample and patterned with 5 *µ*m lines and 5 *µ*m spaces with photolithography and reactive ion etching using 02 plasma (02 flow rate of 100 sccm, chamber pressure of 100 mTorr, 150 W RF power for 25 seconds). The sample was spin coated with poly(methylmethacrylate) (PMMA; A4, Microchem; about 300 nm) at 3000 rpm for 30 seconds and was then immersed in Ni etchant for 30 minutes. The sample was transferred onto low-modulus PDMS (Sylgard 527, Dow Corning; part A to B in a ratio of 1:2; thickness of 600 *µ*m) and dried in an oven at 70°C for 10 minutes. Finally, the PMMA was removed with acetone. The elastic modulus of the sample was measured by a digital force meter (Mark-10). Electrical measurements by impedance and temperature sensors were carried out by an electrochemical workstation (CHI660E, CH Instrument) and a parameter analyzer (B1500A, Agilent), respectively, which were connected with anisotropic conductive films (Son, D. et al. Multifunctional wearable devices for diagnosis and therapy of movement disorders. Nat. Nanotechnol. 9, 397-404 (2014)).

In the present example, a soft electronically instrumented cell-culture platform which includes stretchable nanomembrane electronic devices and patterned graphenenanoribbon aligners was fabricated on a biocompatible low-modulus (about 36.2 kPa) polydimethylsiloxane (PDMS) surface (Figs. 1a and 1b). The low-modulus PDMS substrate offers optimal softness that is compatible with native skeletal muscle tissues. Stretchable physiology (impedance and temperature) sensors based on serpentine buckled gold nanomembranes were fabricated using photolithography (Figs. 2a and 2b). Anisotropically micropatterned (about 5*µ*m lines and spaces) graphene nanoribbons promote cell adhesion, align cells and make proliferation and differentiation easy. When adding gold nanomembranes and graphene nanoribbons on PDMS, the modulus of the system increases slightly by from 36.2 kPa to 42.1 kPa. This stretchable instrumented cell-culture platform has four major functions (Fig. 1c): (i) aligning cells on patterned graphene nanoribbons to promote muscle cell differentiation, (ii) real-time physiological monitoring of cells during proliferation and differentiation by integrated sensors, (iii) serving as an in vitro muscle-on-a-chip platform to test novel nanomaterials or drugs, and (iv) in vivo transfer printing of cell layers for scaffold-free cell sheet treatments.

### Example2. Measurement of the Protein Interaction Force of Graphene

All data describing the adhesion forces between fibronectin molecules and graphene were obtained by an atomic force microscope (AFM) (Dimension Icon, Bruker). For functionalization of the AFM tip with fibronectin, the tip was cleaned with a UV/ozone cleaner (Yuil Ultra Violet System) for 3 minutes, and additionally cleaned with acetone. Next, the tip was soaked in fibronectin solution (5 *µ*g/mL in distilled water; Invitrogen) and completely dried at room temperature. Spring constants of cantilevers (ScanAsyst-Air, Bruker probes) were calibrated using the thermal tune function, and all AFM experiments were conducted in PeakForce QNM mode.

### Example3. Cell Culture

C2C12 myoblasts (CRL-1772; ATCC) and human dermal fibroblasts (PCS-201-012; ATCC) were used for experiments. The C2C12 myoblasts were cultured in two types of media: proliferation medium composed of Dulbecco's modified Eagle's medium (DMEM) supplemented with 10% fetal bovine serum (FBS, Gibco) and 1% penicillin_streptomycin (PS, Gibco); and differentiation medium (to induce myotube formation) composed of DMEM supplemented with 5% horse serum (HS, Gibco) and 1% PS. Human dermal fibroblasts were cultured in DMEM supplemented with 10% FBS and 1% PS. The cells were cultured under the standard culture conditions of 37°C and 5% CO₂. Before culturing the cells on the sample, the fabricated sample was sterilized with 70% ethanol and exposed to UV. The cells were seeded onto the cell-culture platform at following different densities according to each experiment: 2 × 10⁴ cells/cm² for observation of cellular behaviors on the patterned graphene nanoribbons (Fig. 3) and measurement of electrical signals of cells (Figs. 7e to 7h), or 5 × 10⁴ cells/cm² to demonstrate the muscle-on-a-chip (Figs. 7i to 7l) and transfer printing of cell sheets (Figs. 9 and 11; cells were plated at 2 × 10⁴ cells/cm² for Fig. 9e).

### Example4. Cellular Characteristics on Patterned Graphene

To observe the effects of the patterned graphene on cellular behaviors, the cells on the cell-culture platform (support body) were stained with a live/dead viability kit (Life Technologies) using 0.5 µM calcein AM and 5 µM ethidium homodimer-1 in growth medium and were observed using a fluorescence microscope (Eclipse Ti, Nikon). To measure the cell alignment ratio on the patterned graphene, various pattern sizes (spacing/blank size: 3/3, 5/5, 10/10, 20/20, 50/50 or 100/100 *µ*m) on the graphene is fabricated on the low-modulus PDMS. The C2C12 myoblasts cultured on the sample was observed by a phase-contrast microscope (CKX41, Olympus) for three days. Four representative images were captured from each group, and the alignment ratio was obtained using Image_Pro Plus Software (Media Cybernetics). To observe the alignment of cytoskeletal actin, the cells were counterstained with rhodamine phalloidine (Life Technologies), and observed under a fluorescence microscope (Eclipse Ti, Nikon). The cultured cells were fixed in 4% paraformaldehyde solution (Sigma-Aldrich) diluted in phosphate-buffered saline (PBS) for 10 minutes and permeabilized in cytoskeleton (CSK) buffer (150mM sucrose, 50mM NaCl, 3mM MgCl2, 50mM Trizma-base and 0.5% Triton X-100, pH 6.8) for 5 minutes. Next, the cells were incubated in block buffer (10% FBS and 0.1% Tween-20 in PBS) for 2 hours to block nonspecific binding sites and were subsequently incubated with paxillin (1:100; Invitrogen) for 1 hour at 37°C. After washing, the samples were counterstained with DAPI and observed under a fluorescence microscope (Eclipse Ti, Nikon).

Fig. 3 presents details of cell alignment by applying the patterned graphene nanoribbons to a tissue-engineering platform (Fig. 3a). The aligned graphene nanoribbons were transferred on a low-modulus PDMS substrate. The sheet resistance was approximately 1 kΩ/sq (measured using a 4-point probe system [CMT-100MP, Advanced Instrument Technology]), and the water contact angle of the graphene nanoribbons was approximately 112.38° (Figs. 4a to 4c; other characteristics are included). The π -π interaction and hydrophobicity of the graphene, 2D carbon nano sheet result in 6-fold stronger protein adhesion than PDMS alone, as shown in the AFM analysis, in which a fibronectin-coated tip was used (Fig. 3a). The strongly adhesive properties of ECM proteins on graphene facilitated the cell adhesion. Three days after the culture, C2C12 myoblasts were preferably adhered and proliferated on the graphene nanoribbons (Fig. 3b; fluorescence microscopic images, stained with calcein AM).

The width and spacing of graphene nanoribbons are important for cell alignment. C2C12 myoblasts were cultured for 3 days on graphene-nanoribbon substrates with different feature sizes (Fig. 5). Phase-contrast microscopy was utilized to monitor cell alignment during the culture (Fig. 3c; results of real-time monitoring by confocal microscopy was shown in Fig. 6). Cells were considered to be aligned along graphene-nanoribbon patterns when the cell orientation was within the range of about -10° to about +10° with respect to the ribbon orientation. Graphene patterns with nanoribbon widths and spacing of about 5 *µ*m or less show higher alignment ratios than those of 10 ∼ 50 *µ*m, while those of 100 *µ*m show almost no alignment (Figure 3d). Fig. 3e shows the cell alignment on graphene nanoribbons in which horizontal (top) and vertical (bottom) patterns are adjacent to each other. Right frame shows its magnified view. C2C12 myoblast differentiation was enhanced by patterned graphene nanoribbons as indicated by myosin heavy chain (MHC) immunostaining (Figs. 3f to 3h). C2C12 myoblasts exhibited larger myotube area and higher alignment ratio when grown on patterned graphene (Fig. 3i) than those grown on bare PDMS or nonpatterned graphene.

### Example5. Observation of myotube formation

To decide the area and alignment ratio of the myotube formed in the differentiated C2C12 cells, the cultured cells were fixed in 4% paraformaldehyde solution, permeabilized in CSK buffer, and blocked in blocking buffer. The cells were then incubated with antimyosin heavy-chain antibodies (1:100 dilution; Abcam), followed by incubation with Alexa Fluor 488 donkey anti-mouse IgG (1:100 dilution; Abcam) for 1 hour at 37 °C. After washing, the cells were counterstained with DAPI and observed under a fluorescence microscope (Eclipse Ti, Nikon). Four representative images were captured from each group, and the area and alignment ratio of the myotube in each image were measured using Image_Pro Plus Software (Media Cybernetics).

Impedance and temperature sensors were also integrated on the low-modulus PDMS substrate, but beneath the patterned graphene nanoribbons, allowing for the in situ monitoring of cellular activities (Fig. 7a). Cell proliferation and differentiation alter the electrochemical environment around electrodes, and these changes can be detected as impedance or conductance changes. For the impedance sensor, the alternating current (AC) flows through the culture medium between two serpentine electrodes spaced apart from each other by 600 *µ*m. For the temperature sensor, a resistance temperature detector based on a serpentine gold nanomembrane was used. Figs. 7b to 7d show the calibration curves of the temperature, impedance, and conductance sensors. Figs. 7e and 7f show changes in impedance during the proliferation and differentiation of C2C12 myoblasts, respectively. The cells on electrodes acted as insulating barriers that impede the contact between electrodes and conductive media, thereby increasing the measured impedance as cells proliferate (Fig. 7e). Formation of myotubes during myogenic differentiation increased conductance and thereby reduced impedance (Fig. 7f). Fig. 7g shows time dependent impedance changes at 17.7 kHz with respect to cell physiology changes. Fibroblasts that do not form tubular structures were used as a control group. Such comparison illustrated that proliferation increases impedance while differentiation (in growth medium or differentiation medium) decreases impedance. The corresponding conductance measurements exhibited opposite trends (Fig. 7h).

### Example6. Preparation of Ceria Nanoparticles

To synthesize ceria nanoparticles, 1 mM (0.4 g) cerium(III) acetate (98%, Sigma-Aldrich) and 12mM (3.2 g) oleylamine (approximate C18 content of 80 ∼ 90%, AcrosOrganics) were added to 15 mL of xylene (98.5%, Sigma-Aldrich). The mixed solution was sonicated for 15 minutes at room temperature and then heated to 90°C. One milliliter of distilled water was injected into the solution under vigorous stirring at 90 °C, and the solution color was changed to an off-white color, which indicates that the reaction had occurred. The resulting mixture was aged for 3 hours at 90 °C to obtain a light yellow colloidal solution, which was then cooled to the room temperature. Acetone (100 mL) was added to the precipitated ceria nanoparticles. The precipitate was washed with acetone using centrifugation, and the resulting ceria nanoparticles were easily dispersed in organic solvents, e.g., chloroform. For the synthesis of phospholipid-PEG-capped ceria nanoparticles, the ceria nanoparticles dispersed in chloroform were encapsulated by a PEG-phospholipid shell. First, 3 mL of ceria nanoparticles in CHCl₃ (10 mg/mL) was mixed with 3 mL of CHCl₃ solution containing 30 mg of mPEG-2000 PE. The solvent was then evaporated using a rotary evaporator and incubated at 70 °C in a vacuum for 1 hour to completely remove the chloroform. The addition of 5 mL of distilled water yielded a clear and light yellowish suspension. After filtration, excess mPEG-2000 PE was removed using ultracentrifugation. Purified phospholipid-PEG-capped ceria nanoparticles were dispersed in distilled water.

### Example7. Application of muscle-on-a-chip

To observe the viability of cells over time, the cells were treated with 5 mM H₂O₂ and/or 0.1 mM Ceria nanoparticles. Next, the cells selected at five different time (0, 300, 600, 900 or 1800 seconds) were cultured in live/dead viability analysis medium (0.5 µM calcein AM and 5 µM ethidium homodimer-1 in growth medium) for 30 minutes and visualized by a fluorescence microscope (Nikon). Four representative images were captured from each group, and the area of survival cells (green color) were measured using Image_Pro Plus Software (Media Cybernetics). All data of electrical signals were collected using an electrochemical workstation (CHI660E, CH Instrument). The sample was disposed at experiment solution (37 °C) and the impedance was measured continuously for 30 minutes. In order to guarantee the reproducibility of each experiment, the experiment on each group was repeated 3 times.

The culture of target tissue on the substrate equipped with electronic sensors and the test of the effect of new materials about this tissue provide useful information for the biological system and prevent the sacrifice of animals. As a demonstration, we, inventors designed a skeletal muscle ischemia model using C2C12 myoblasts. Ischemia-induced reactive oxygen species (ROS) decrease cell viability and cause inflammation. Therefore, scavenging ROS is important under this system. Several nanomaterials and molecules have been proposed to suppress ROS generation (Karakoti, A., Singh, S., Dowding, J.M., Seal, S. & Self, W.T. Redox-active radical scavenging nanomaterials. Chem. Soc. Rev. 39, 4422-4432 (2010)). Ceria nanoparticles are an attractive candidate for this purpose (Fig. 7i). The relevant concentrations of hydrogen peroxide (H₂O₂) and ceria nanoparticles were optimized as shown in Figs. 8a to 8c. Then, C2C12 myoblasts were exposed to 5mM H₂O₂ to simulate ischemia-induced ROS, and fluorescence images (Fig. 7j) were obtained to analyze the applicability of ceria nanoparticles. The use of ceria nanoparticles improved cell viability, and quantitative comparison of living cells (Fig. 7k) confirmed the effects of ceria nanoparticles on ROS scavenging.

The cell viability, represented by the impedance of the culture, was also monitored by the instrumented cell-culture platform (Fig. 7l). Treatment with 5 M H₂O₂ in differentiated C2C12 cells caused instant death in the majority of cells, resulting in a sudden, dramatic increase in impedance. Then impedance slowly decreased over 30 minutes as dead cells gradually are detached from the substrate and conductive media flows between dead cells and electrodes. Treatment with 5 mM H₂O₂ induced similar but slower changes in impedance. However, treatment with 5mM H₂O₂ in the presence of ceria nanoparticles yielded only minimal changes in impedance.

### Example8. Transfer Printing of C2C12 Cell Sheets

The cell-cultured platform including the cultured C2C12 myoblasts was disposed on tissue culture polystyrene so as to be upside down by conformal contact for 30 seconds. The cell-cultured platform was peeled off slowly from the tissue culture polystyrene, and then by gently tapping the upper side of the platform for a few seconds by fingers, it was disposed on a desired substrate so as to be upside down by conformal contact. After 90 seconds, the platform was peeled off slowly beginning with an edge, and then the cell sheet was transferred onto the substrate. In order to visualize the transferred cell sheet, the cell sheet transferred on the substrate was stained with hematoxylin solution (Mayer's hematoxylin solution, ScyTek). To measure the viability of cells after the transfer printing, the cells were stained with a live/dead viability kit (Life Technologies) of 0.5 mM calcein AM and 5 mM ethidium homodimer-1 in growth medium and observed by a fluorescence microscope (Nikon). To decide the transfer yield, the ratio of the area of the transferred cell sheet to the area of the cell sheet on the platform before the transfer was calculated using Image_Pro Plus Software (Media Cybernetics). To form multilayered cell sheet, three samples of cell sheets were prepared on the cell-culture platform, where each cell sheet was previously stained with fluorescence dye, colored with DiD (red; two samples; Invitrogen) or DiO (green; one sample; Invitrogen). Each cell sheet was transferred in the order of red, green, red, and sheets were stacked on top of each other. The arrangement of multilayered cell sheets was observed using a confocal microscope (TCS SP8 STDE, Leica). To clearly visualize each cell sheet, the color intensities of the captured images were modified using the Leica Application Suite (Leica).

Cell sheets cultured and differentiated on the cell-culture platform can be applied to in vivo therapies via scaffold-free localized treatments. Through continuous monitoring of cells during the preparation of cell sheets, the cell condition and degree of differentiation can be confirmed before the application of the cell sheets to biological systems which can be carried out by using the transfer printing. The transfer printing process of the cell sheet from the cell-culture platform to the receiving substrate (protein-coated glass) is shown in Fig. 9a. The complete process consists of two steps, preparation (on tissue culture polystyrene for 30 seconds) and transfer (on the receiving substrate for 90 seconds) steps. The well-structured configuration and high viability of the transferred C2C12 cell sheet were observed by staining cells with hematoxylin and conducting viability assays (Fig. 9b and inset). Differentiation characteristics were also maintained after the transfer printing (Fig. 9c). Figs. 9d and 9e represent the effects of contact time during the transfer step (changes in cell-to-protein adhesion) and cell culture time (changes in cell density) on transfer yields, respectively. As illustrated in Fig. 10, high transfer yields (about 90%) were obtained with contact time longer than 90 seconds and culture time longer than 6 days. Figure 9f shows a large-area (5 cm × 5 cm) cell sheet transfer, which is critical for human applications, and Figs. 9g and 9h provide a schematic illustration of a triple-layered cell sheet prepared using multiple transfer printings and confocal microscope images of these sheets.

Fig. 10 shows a simplified theoretical explanation for the cell sheet transfer printing. In view of fracture mechanics, the crack propagation criterion on an interface is G = Γ, where G is the energy release rate (that is, the strain energy dissipated when the cracked interface advanced per a unit area of newly cracked surface) and Γ is the interface toughness. As both graphene and cell sheets are much thinner than the PDMS substrate, they both can be overlooked when computing G because they store negligible strain energy. Therefore, we, inventors simplify the cell sheet transfer printing problem to a 2D plane strain model of PDMS on glass, as illustrated by the schematic in Fig. 10a, with PDMS thickness h, interfacial crack size a, and vertical peel force F. When considering the glass as a rigid substrate, analytical and finite element modeling (FEM) results about the G(F, a) relation are shown in the graph of Fig. 10a. Both results indicate that G increases monotonically with both a and F.

Fig. 10b explains why the cell sheet cannot be directly transferred to protein-coated glass. Graphene and the cell sheet are intentionally made visible in Fig. 10b. Cells aggregate on grephene so that the edge of the PDMS is not covered by graphene, and thus cells are considered to be pre-delaminated from the glass substrate having an initial crack size a₀ identical to the margin size. When assuming F_{c/graph} > Γ_{c/glasss}, the peel force needed to delaminate the cell-glass interface (F₁^{D}) is smaller than that for the cell-graphene interface (F₂^{D}), suggesting that the cell sheet is easier to be delaminated along the cell-glass interface and thus cannot be transferred to the glass substrate successfully.

The outcome will be different if the preparation step shown in Fig. 10c is taken before transferring the cell sheet to protein-coated glass. When assuming Γ_{c/graph} > F_{c/polystyrene}, the cell sheet will not be transferred onto polystyrene during the preparation step for the same reason as explained in Fig. 10b. However, edge damage can be found in the cell sheet during the delamination step, as evidenced by the phase-contrast microscope images in Fig. 10d. Damaged cells near the edge as living matter tend to contract or roll up, and lead to interfacial shear crack between the cell sheet and graphene, as highlighted by the red line in Fig. 10e. Hence, when conducting the final transfer printing after the preparation step, the new crack lengths become a₁^{F} and a₂^{F}, and most importantly, a₂^{F} is large enough in comparison with a₁^{F}. As a result, F₂^{F} is smaller than F₁^{F} (F₂^{F} < F₁^{F}), which indicates that the cell-graphene interface will delaminate with a smaller peel force. Therefore, the cell sheet can be successfully transferred onto protein-coated glass.

When there is no PDMS margins, i.e., when the PDMS surface is fully covered by graphene, the edge damage of cells decreases significantly during the preparation step. Therefore, as shown in Fig. 10f, a₂^{M} will be only slightly larger than a₁^{M}. According to the graph in Fig. 10e, F₁^{M} < F₂^{M}, and hence, the cell-glass interface will first delaminate. This hypothesis is validated by the experimental results shown in the right panel of Fig. 10f, and it explains why maintaining reasonable PDMS margin size is critical for the success of cell sheet transfer.

### Example9. Mouse Scarred Muscle Model

All experiments on animals were approved by the animal care committee at Seoul National University Hospital. Six-week-old male BALB/c nude mice were anesthetized by intraperitoneal injection with a mixture of zolazepam and xylazine. A surgical skin incision (1.5 cm) was made in the ventral aspect of the right thigh beginning near the groin. Subsequently, the medial thigh muscle was dissected 5 mm proximal to and along the course of the deep femoral branch. The dissection continued laterally 5mm from and along the bundle of the femoral nerve, artery, and vein. The entire medial thigh muscle was excised. The surgical area was evaluated by micro computed tomography (Micro-CT).

After the cell sheet transplantation (1 layer, 5 layers or none; each group N = 4) to the scarred muscles in the hindlimb, the therapeutic effect was monitored over time by micro-CT. The micro-CT was performed using a cone-beam-type commercial preclinical CT scanner (NFRPolaris-G90C, NanoFocusRay) with the following imaging parameters: tube voltage, 50 kVp; tube current, 100 µA; field of view, 35 mm × 35 mm; matrix, 512 × 512; slice thickness, 0.054 mm. Three-dimensional reconstructed images were obtained using OsiriX (version 4.0, 32 bit, OsiriX Foundation).

For fluorescent imaging, Balb/c nude mice (female, ages 6 ∼ 8 weeks) were subjected to operation with cell tracer (DiD)-labeled GFP-expressing cell sheets and anesthetized by intraperitoneal injection. Imaging of mice was carried out with an IVIS Spectrum instrument (IVIS, PerkinElmer) in epifluorescence mode equipped with 644 and 665 nm filters for excitation and emission, respectively, from 1 day after transplantation. Measurements of the fluorescence signal from each cell sheet were made using Living Image Software 4.0 (Perkin Elmer).

All tissues were embedded in paraffin and sectioned to 4 *µ*m thickness. Histology samples for morphology visualization were stained by hematoxylin and eosin using standard protocols. For immunofluorescence staining, the prepared paraffin sections were dewaxed, hydrated and treated with 0.01% protease XXIV (Sigma-Aldrich) in PBS for 20 minutes at 37 °C. The specimens were then stained with primary anti-GFP antibodies (Santa Cruz Biotechnology) according to the manufacturer's instructions. Protein expression was visualized with Alexa Fluor 488-conjugated secondary antibodies (Invitrogen). All images were acquired using an inverted fluorescence microscope (DM5500 B, Leica). In order to detect vascularization in organized tissues from the cell sheet, immunohistochemistry was performed using anti-CD31 antibodies (Novus Biologicals) of following step. First, endogenous peroxidase and protein were blocked with a solution of 0.3% H₂O₂ and goat serum (Dako) to prevent nonspecific antibody binding. After 30 minutes, the tissues were incubated with the primary anti-CD31 antibody for 1 hour, washed, and incubated with horseradish peroxidase-conjugated goat anti-rabbit secondary antibodies (Santa Cruz Biotechnology) for 1 hour. After additional washes, vascularization was evaluated by staining with 3,3'-diaminobenzidine (DAB) peroxidase substrate (Dako).

To investigate the therapeutic effects of in vivo cell sheet transfer printing on scarred muscles in mice, we, inventors used green fluorescent protein (GFP)-transfected C2C12 myoblasts and imaging modalities to evaluate tissue organization. We, inventors grafted monolayer or five-layered C2C12 cell sheets onto the scarred area on the hindlimbs of mice (Fig. 11a). The temperature changes during the cell sheet transfer printing were measured by the temperature sensor of the instrumented cell-culture platform. Fast transfer printing (about 90 seconds) causes minimal body temperature changes (about 1.5 °C).

Transplanted cell sheets were labeled with a cell tracer (DiD) and monitored over time by measuring the fluorescence. Fluorescence was detected on the scarred area, even after 7 days, while no signal was observed in the opposite hindlimb sites (see Fig. 11b regarding fluorescence data of five-layered cell sheet). Fluorescence microscope images of sectioned tissue slices from transfected sites show the growth of the transplanted five-layered cell sheets 7 days after the transfer printing (Fig. 11c), which is consistent with in vivo fluorescence imaging results. The capability to maintain the organization and function of a transplanted cell sheet can maximize the therapeutic effect of localized cell therapy at the wound site.

In addition, micro-computed tomography (micro-CT) also confirms the growth of muscular tissues in the transplanted region (Fig. 11d). The white arrows indicate the operation site. Comparison between the first and second frames of Fig. 11d highlights removed muscle tissues. Importantly, 7 days after transplantation of the cell sheet, muscle tissues were regenerated (Fig. 11d, third frame), while no recovery was observed without cell sheet transfer after the surgery (Fig. 11d first frame; the inset shows the preoperation image of the negative experiment group). Hematoxylin and eosin staining images of low magnification rate reveal that five-layered cell sheets have grown at transferred sites (Fig. 11c). On the other hand, atrophic changes in muscles occured in the scarred muscles without cell sheet transplantation (Fig. 11f). The monolayer cell sheet also showed therapeutic effects on the scarred region. As shown in Figs. 11g and 11h, the abundant proliferation and differentiation of C2C12 myoblasts in five-layered cell sheets transferred into scarred muscle sites were observed through the detection of GFP expression. Vascularization was also observed by CD31 staining (Fig. 11i).

Although the embodiments of the present invention have been disclosed for illustrative purposes, those skilled in the art will appreciate that the present invention may be embodied in other specific ways without changing the technical spirit or essential features thereof. Therefore, the embodiments disclosed in the present invention are not restrictive but are illustrative. The scope of the present invention is given by the claims, rather than the specification, and also contains all modifications within the meaning and range equivalent to the claims.

### Industrial Applicability

By fabricating the cell sheet using the device of the present invention, the physiological change of cells which could be detected through various complicate methods can be detected easily and in real time by electrical signals, and this real time measuring method guarantees the ease and accuracy of the experiment. Moreover, by measuring electrical signals, there is an advantage to block cellular loss resulting from prior experiment methods.

In addition, since it is possible to perform the transfer printing of the cell sheet under at optimal state by observing cells state in real time, the treatment effect can be maximized. The regeneration of damaged tissues can be maximized by performing the transfer printing of the activated cell sheet fabricated in this way to the desired body tissue through newly developed transfer printing method with high yield near 100 %.

## Claims

1. A cell sheet manufacturing device comprising:
a support layer;
a patterned electrode formed adjacent to the support layer; and
a graphene layer formed adjacent to the electrode.

2. The cell sheet manufacturing device of claim 1, wherein the support layer is formed of silicon rubber, glass, PET film or wafer.

3. The cell sheet manufacturing device of claim 2, wherein the silicon rubber is polydimethylsiloxane.

4. The cell sheet manufacturing device of claim 1, wherein the electrode is an electrode selected from a group consisting of Al, Cu, Pt and Cr/Au.

5. The cell sheet manufacturing device of claim 1, wherein the graphene layer is a patterned layer.

6. The cell sheet manufacturing device of claim 1, wherein a state of cell growth and proliferation can be checked in real time from an electric signal obtained through the electrode.

7. A manufacturing method of a cell sheet manufacturing device comprising:
(i) forming a nickel layer on a silicon wafer;
(ii) forming an electrode layer on the nickel layer in order;
(iii) patterning the electrode layer;
(iv) forming a graphene layer on the patterned electrode layer;
(v) forming an adhesion layer on the graphene layer;
(vi) separating the silicon wafer by etching and removing the nickel layer;
(vii) attaching the separated adhesion layer/graphene layer/electrode layer on silicon rubber layer; and
(viii) removing the adhesion layer.

8. The manufacturing method of a cell sheet manufacturing device of claim 7, wherein the electrode layer is an electrode layer selected from a group consisting of Al, Cu, Pt and Cr/Au.

9. The manufacturing method of a cell sheet manufacturing device of claim 7 further comprising patterning the graphene layer before the step (v).

10. The manufacturing method of a cell sheet manufacturing device of claim 7, wherein the adhesion layer is PMMA.

11. The manufacturing method of a cell sheet manufacturing device of claim 10, wherein the PMMA is PMMA A2 or PMMA A4.

12. A manufacturing method of a cell sheet comprising culturing cells on a cell sheet manufacturing device which comprises a support layer, a patterned electrode formed adjacent to the support layer and a graphene layer formed adjacent to the electrode.

13. The manufacturing method of a cell sheet of claim 12, wherein the support layer is formed of silicon rubber, glass, PET film or wafer.

14. The manufacturing method of a cell sheet of claim 13, wherein the silicon rubber is polydimethylsiloxane.

15. The manufacturing method of a cell sheet of claim 12, wherein the electrode is an electrode selected from a group consisting of Al, Cu, Pt and Cr/Au.

16. The manufacturing method of a cell sheet of claim 12, wherein the graphene layer is a patterned layer.

17. The manufacturing method of a cell sheet of claim 12, wherein a state of cell growth and proliferation can be checked in real time from an electric signal obtained through the electrode.
